# EUROPÄISCHE PATENTSCHRIFT

(11) **EP 0 504 799 B1**
(45) Veröffentlichungstag und Bekanntmachung des Hinweises auf die Patenterteilung: **02.07.1997**
(21) Anmeldenummer: 92104584.5
(22) Anmeldetag: 17.03.1992
(51) Int. Cl.: C07D 213/81, A61K 31/44

(54) **Gemischte Pyridin-2,4- und 2,5-dicarbonsäurediamide, Verfahren zu ihrer Herstellung, Verwendung derselben sowie Arzneimittel auf Basis dieser Verbindungen**
Mixted pyridine-2,4- and 2,5-dicarboxylic acid diamides, process for their preparation, their use as well as medicaments based on these compounds
Diamides mixtes d'acides pyridine-2,4- et 2,5-dicarboxyliques, procédé pour leur préparation, leur utilisation ainsi que médicaments basés sur ces composés

(30) Priorität: 18.03.1991 DE 4108824
(43) Veröffentlichungstag der Anmeldung: 23.09.1992
(73) Patentinhaber: HOECHST AKTIENGESELLSCHAFT, 65926 Frankfurt am Main (DE)
(72) Erfinder: Baader, Ekkehard, Dr., W-6240 Königstein/Taunus (DE); Bickel, Martin, Dr., W-6380 Bad Homburg (DE); Günzler-Pukall, Volkmar, Dr., W-3550 Marburg (DE); Volz, Manfred, Dr., W-6390 Usingen (DE)

(56) Entgegenhaltungen:
- EP-A- 0 278 453
- EP-A- 0 353 668
- EP-A- 0 409 119
- EP-A- 0 414 216
- EP-A- 0 430 029

## Beschreibung

Verbindungen, die die Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von der Zelle nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren wie α,α'-Dipyridyl zu einer Hemmung der C1_{q}-Biosynthese von Makrophagen führt (W. Müller et al., FEBS Lett. 90 (1978), 218; Immunbiology 155 (1978) 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß Prolinhydroxylase durch Pyridin-2,4 und -2,5-dicarbonsäure effektiv gehemmt wird (K. Mayama et al., Eur. J. Biochem. 138 (1984) 239-245). Diese Verbindungen sind in der Zellkultur allerdings nur in sehr hohen Konzentrationen als Hemmstoffe wirksam (Tschank, G. et al. Biochem. J. 238 (1987) 625 - 633).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und-2,5-dicarbonsäure, die die Kollagenbiosynthese im Tiermodell wirksam hemmen.

So wird in der DE-A 37 03 959 unter anderem die Synthese von N,N'-Bis(2-methoxyethyl)-pyridin-2,4-dicarbonsäurediamid und N,N'-Bis(3-isopropoxypropyl)-pyridin-2,4-dicarbonsäurediamid beschrieben.

In den deutschen Patentanmeldungen P 38 26 471.4 und P 38 28 140.6 wird ein verbessertes Verfahren zur Herstellung von N,N'-Bis(2-methoxyethyl)-pyridin-2,4-dicarbonsäurediamid vorgeschlagen. Die deutsche Patentanmeldung P 3924093.2 schlägt neue N,N'-Bis(alkoxyalkyl)-pyridin-2,4-dicarbonsäurediamide vor.

Es war deshalb die Aufgabe zu lösen, Verbindungen zu finden, die in weit besserer Weise als die bisher bekannten zur Inhibierung der Prolin- und Lyrinhydroxylase geeignet sind. Gelöst wurde die Aufgabe durch Pyridin-2,4 und 2,5-dicarbonsäurediamide der Formel I 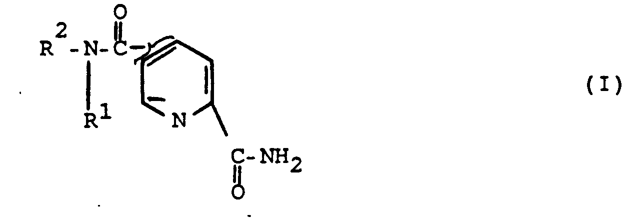 worin
- R¹: C₁-C₁₂-Alkyl bedeutet, welches unsubstituiert oder einfach oder im Falle der C₂-C₁₂-Alkyle auch mehrfach substituiert ist mit
Phenyl, Hydroxy, Alkoxy, Amino, Alkoxycarbonyl, Alkyl- oder Dialkylamino, wobei die Alkylreste 1-3 C-Atome aufweisen,
- oder: R¹ Phenyl bedeutet,
- oder: R¹ unter der Voraussetzung, daß R² H ist, Amino bedeutet, welches unsubstituiert ist oder mono-substituiert ist mit Methylcarbonyl
und
- R²: Wasserstoff bedeutet,
- oder: wobei die Reste R¹ und R² zusammen mit dem Stickstoffatom einen Rest der Formel 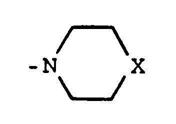 bilden,
worin
- X: 0, CH₂ oder N-R³ bedeutet,
wobei
- R³: Wasserstoff oder Methyl bedeutet, sowie die physiologisch verträglichen Salze.

"Mehrfach substituiert" bedeutet im Vorstehenden und Folgenden, daß mindestens 2, höchstens 4 in den Alkylresten vorhandenen Wasserstoffatome durch die genannten Substituenten ersetzt sind. Bei Mehrfachsubstitutionen können die Substituenten auch voneinander unabhängig verschieden sein.

Alle genannten Alkylreste mit mehr als 2-C-Atomen und alle Alkinylreste mit mehr als 3-C-Atomen können sowohl geradkettig als auch verzweigt sein.

Weiterhin betrifft die Erfindung die Verbindungen der Formel I zur Anwendung als Arzneimittel. Außerdem betrifft die Erfindung die Verbindungen der Formel I zur Anwendung als Fibrosuppressiva und Immunsuppressiva sowie zur Inhibierung der Prolin- und Lysinhydroxylase und zur Beeinflussung des Stoffwechsels von Kollagen und kollagenähnlichen Stoffen bzw. der Biosynthese von C1q. Inhibitoren der Prolylhydroxylase sind geeignete Werkzeuge in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma) sowie der Atherosklerose.

Die ausschließlich in Position 4 bzw. 5 in der Amidgruppe substituierten Pyridin-2,4 und -2,5-dicarbonsäurediamide der allgemeinen Formel I zeigen zum Vergleich zu den in Position 5 auch mit Carbonsäureamidgruppen substituierten Pyridin-2,4-dicarbonsäurediamide aus der DE-A-3 707 429 und im Vergleich zu den in beiden Amidgruppen substituierten Pyridin-2,4 und -2,5-dicarbonsäurediamide der DE-A-37 039 59 ohne wesentliche und überraschende verbesserte Wirksamkeit bei der Hemmung der Prolin- und Lysinhydroxylase im Tierversuch.

Weiterhin betrifft die Erfindung ein Verfahren zur Herstellung von Verbindungen der Formel I, das dadurch gekennzeichnet ist, daß man
eine Verbindung der Formel II' 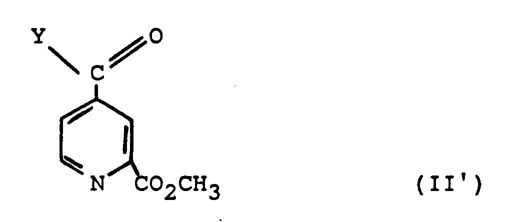 mit einer Verbindung der Formel III 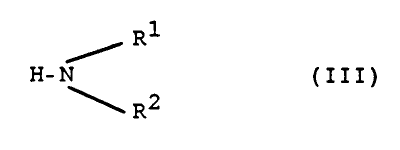 umsetzt, wobei R¹ und R² die zu Formel I angegebenen Bedeutungen haben und Y Halogen, insbesondere Chlor ist
und anschließend die entstehende Verbindung der Formel IV 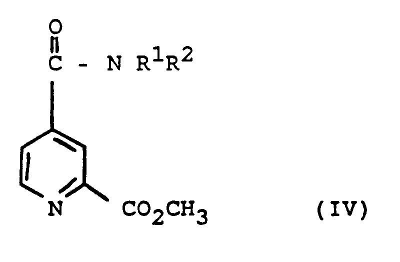 mit NH₃ in eine Verbindung der Formel I 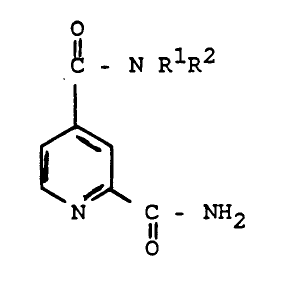 überführt und gegebenenfalls in ihre physiologisch verträglichen Salze überführt.

Das folgende Reaktionsschema zeigt den Herstellungsweg (Stufen 5 und 6), einschließlich der Synthese der Vorstufen (1 bis 4)

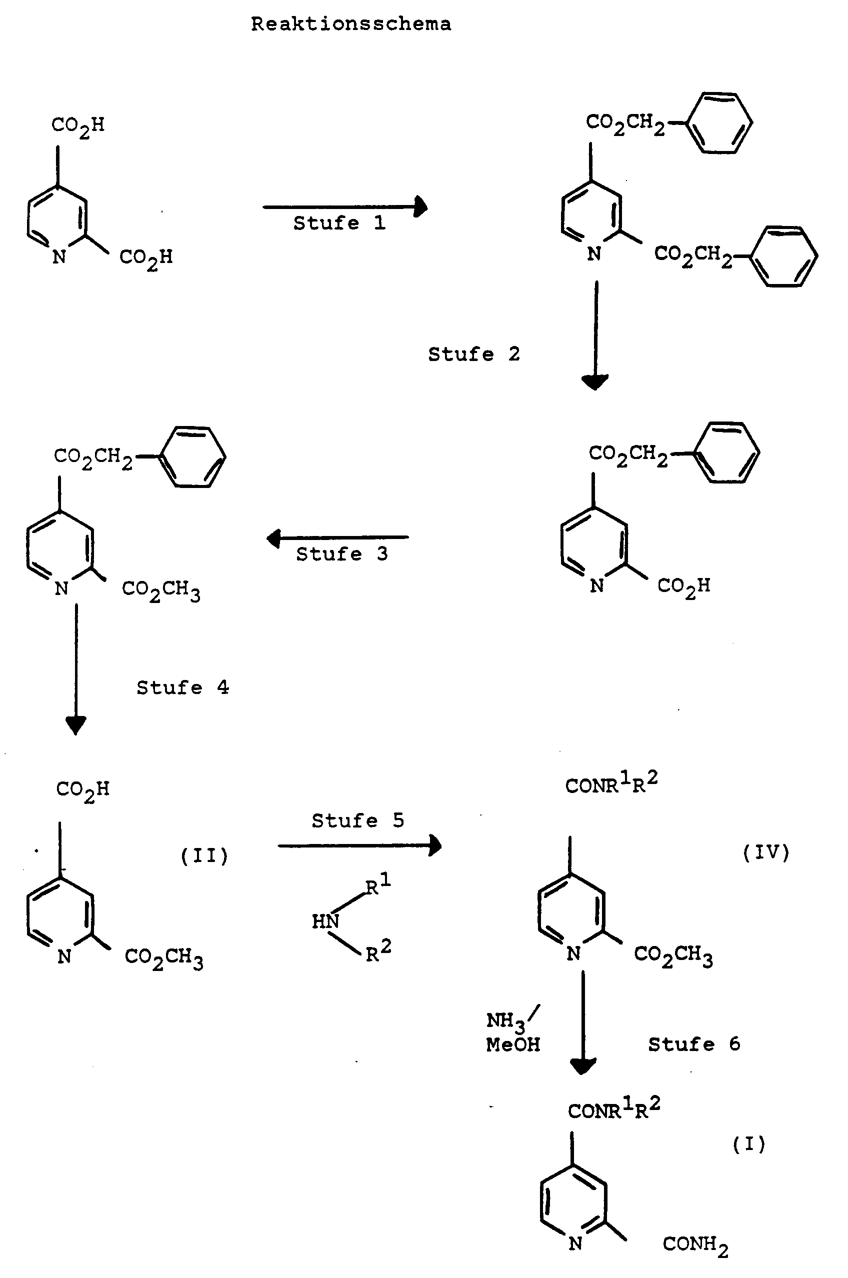

Gemäß Stufe 1 wird die käufliche Pyridin-2,4-dicarbonsäure in ihr Dicarbonsäuredihalogenid, vorzugsweise ihr -dichlorid, überführt und mit einem gegebenenfalls substituierten Benzylalkohol zum Pyridin-2,4-dicarbonsäuredibenzylester umgesetzt.

Gemäß Stufe 2 wird der Diester selektiv in 2-Stellung z. B. in Gegenwart eines Kupfer-Salzes gemäß Delarge, J.: Phar. Acta. Helv. 44 (10), 637 (1969) verseift.

Die freie Säurefunktion in 2-Stellung wird anschließend in Stufe 3 in das entsprechende Säurechlorid überführt und mit einem Alkohol, wie z. B. Methyl- oder Ethylalkohol in den entsprechenden 2-Carbonsäureester umgesetzt.

Die verbliebene Benzylschutzgruppe in 4-Stellung wird gemäß Stufe 4 hydrogenolytisch abgespalten (z. B. mit H₂/Pd, Houben-Weyl: Bd. IV/1c (1980), S. 381 - 82).

Die freie Säure in 4-Stellung (Formel II) wird in ihr Säurehalogenid, vorzugsweise -chlorid, umgewandelt. Das Säurechlorid kann nun mit dem Amin der allgemeinen Formel (III) in den gemischten Pyridin-4-carbonsäureamid-2-carbonsäureester (IV) übergeführt werden.

Mit alkoholischer Ammoniaklösung (z. B. in Methanol) wird aus dem 2-Carbonsäureester (IV) das gemischte Diamid der Formel (I) hergestellt.

Das genannte Verfahren, das gemäß dem Reaktionsschema für die in 4-Position substituierten Verbindungen beschrieben worden ist, gilt auch für die Verbindungen, die in 5-Position entsprechend substituiert sind.

Gegebenenfalls kann die Aufarbeitung der Produkte beispielsweise durch Extraktion oder durch Chromatographie z.B. über Kieselgel erfolgen. Das isolierte Produkte kann umkristallisiert und gegebenenfalls mit einer geeigneten Säure zu einem physiologisch verträglichen Salz umgesetzt werden. Als geeignete Säuren kommen beispielsweise in Betracht:
Mineralsäuren, wie Chlorwasserstoff- und Bromwasserstoffsäure sowie Schwefel-, Phosphor-, Salpeter- oder Perchlorsäure oder organische Säuren wie Ameisen-, Essig-, Propion-, Bernstein-, Glykol-, Milch-, Äpfel-, Wein-, Zitronen-, Malein-, Fumar-, Phenylessig-, Benzoe-, Methansulfon-, Toluolsulfon-, Oxal-, 4-Aminobenzoe-, Naphthalin-1,5-disulfon- oder Ascorbinsäure.

Die Ausgangsverbindungen der Formel (III) können, soweit sie nicht käuflich sind, einfach synthetisiert werden (z.B. Organikum, Organisch Chemisches Grundpraktikum, 15. Aufl., VEB Deutscher Verlag der Wissenschaften, 1976; eine Übersicht über die verschiedenen Möglichkeiten findet sich im Methodenregister, S. 822).

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum und Immunsuppressivum.

Die Aktivität der Fibrogenase kann durch radioimmunologische Bestimmung des N-terminalen Propeptiids des Kollagens Typ-III oder der N- bzw. C-terminalen Quervernetzungsdomäne des Kollagens Typ-IV (7s-Kollagen bzw. Typ-IV-Kollagen-NC₁) im Serum bestimmt werden.

Zu diesem Zweck wurden die Hydroxyprolin-, Prokollagen-III-Peptid-, 7s-Kollagen- und Typ-IV-Kollagen-NC₁-Konzentrationen in der Leber von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, S. 335-476, New York, Academic Press, 1964).

Aufgrund dieser pharmakologischen Eigenschaften sind die erfindungsgemäßen Verbindungen zur Behandlung von Störungen des Stoffwechsels von Kollagen und kollagenähnlichen Stoffen, bzw. zur Behandlung von Störungen der Biosynthese von Clq geeignet.

Die Erfindung betrifft daher weiter die Anwendung der erfindungsgemäßen Verbindungen der Formel I sowie deren physiologisch verträglichen Salze bei der Behandlung der oben genannten Stoffwechselstörungen.

Die Verbindungen können entweder allein oder mit physiologisch verträglichen Hilfs- oder Trägerstoffen vermischt als Arzneimittel angewendet werden. Sie können zu diesem Zweck oral in Dosen von 0,01 - 25,0 mg/kg/Tag, vorzugsweise 0,01 - 5,0 mg/kg/Tag oder parenteral in Dosen von 0,001 - 5 mg/kg/Tag, vorzugsweise 0,001 - 2,5 mg/kg/Tag, insbesondere 0,005 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimittel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen,

Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral oder rektal appliziert werden.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen. Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden.

Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage, physiologische Kochsalzlösung oder Alkohole, z. B. Ethanol, Propanol, Glycerin, daneben auch Zuckerlösungen wie Glukose- oder Mannitlösungen, oder auch eine Mischung aus den verschiedenen genannten Lösungsmitteln.

Nachfolgend ist die Erfindung an Hand von Beispielen näher erläutert.

### Allgemeine Vorschrift zur Herstellung der Verbindungen

1 mmol Pyridin-4-Carbonsäureamid-2-carbonsäuremethylester (IV) werden in 30 ml gesättigter methanolischer Ammoniak-Lösung gelöst und 2 Stunden bei Raumtemperatur gerührt. Die Lösung wird eingeengt und der Rückstand mit Diisopropylether verrührt und abgesaugt.

### Beispiel 1

Pyridin-4-carbonsäure-ethylamid-2-carbonsäureamid
Fp.: 197° C

### Beispiel 2

Pyridin-4-carbonsäure-N-morpholinamid-2-carbonsäureamid
Fp.: 128° C

### Beispiel 3

Pyridin-4-carbonsäure-diethylamid-2-carbonsäureamid
Öl, MS = 222 (M + H⁺) Molmasse C11H15N302 (221)

### Beispiel 4

Pyridin-4-carbonsäure-(2-methoxyethyl)-amid-2-carbonsäureamid
Fp.: 116 - 120° C

### Beispiel 5

Pyridin-4-carbonsäure-(3-methoxypropyl)-amid-2-carbonsäureamid
Fp.: 149° C

### Beispiel 6

Pyridin-4-carbonsäure-(3-hydroxypropyl)-amid-2-carbonsäureamid
Fp.: 154 - 156° C

### Beispiel 7

Pyridin-4-carbonsäure-(alanyl)-amid-2-carbonsäureamid
Fp.: 124 - 125° C

### Beispiel 8

Pyridin-4-carbonsäure-(O-benzylalanyl)-amid-2-carbonsäureamid
Fp.: 138 - 140° C

## Patentansprüche (Patentansprüche für folgende(n) Vertragsstaat(en): AT, BE, CH, DE, DK, FR, GB, IT, LI, LU, NL, PT, SE)

1. Pyridin-2,4- und 2,5-dicarbonsäurediamide der Formel I 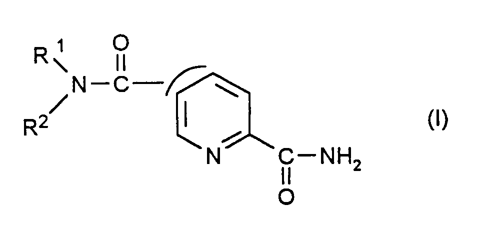 worin
R¹ C₁-C₁₂-Alkyl, bedeutet, welches unsubstituiert oder einfach oder im Falle der C₂-C₁₂-Alkyle auch mehrfach substituiert ist mit
Phenyl, Hydroxy, Alkoxy, Alkoxycarbonyl, oder Dialkylamino, wobei die Alkylreste 1 bis 3 C-Atome aufweisen,
oder R¹ Phenyl bedeutet,
oder R¹ unter der Voraussetzung, daß R² H ist, Amino bedeutet, welches unsubstituiert ist oder mono-substituiert ist mit Methylcarbonyl
und
R² Wasserstoff bedeutet,
oder wobei die Reste R¹ und R² zusammen mit dem Stickstoffatom einen Rest der Formel 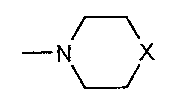 bilden,
worin
X O, CH₂ oder N-R³ bedeutet,
wobei
R³ Wasserstoff oder Methyl bedeutet, sowie die physiologisch verträglichen Salze.

2. Verfahren zur Herstellung von Verbindungen der Formel I nach Anspruch 1, dadurch gekennzeichnet, daß man eine Verbindung der Formel II' 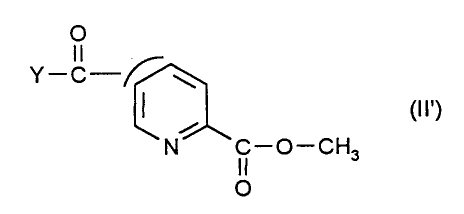 mit einer Verbindung der Formel III, 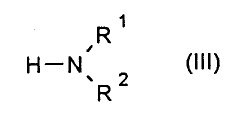 umsetzt, wobei R¹ und R² die zu Formel I angegebenen Bedeutungen haben und Y Halogen, insbesondere Chlor ist, und anschließend die entstehende Verbindung der Formel IV 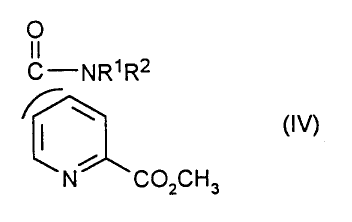 mit NH₃ in eine Verbindung der Formel I und in ihre physiologisch verträglichen Salze überführt.

3. Verbindungen nach Anspruch 1 zur Anwendung als Arzneimittel.

4. Verbindungen nach Anspruch 1 zur Inhibierung der Prolin- und Lysinhydroxylase.

5. Verbindungen nach Anspruch 1 zur Anwendung als Fibrosuppressiva und Immunsuppressiva.

6. Arzneimittel, enthaltend eine Verbindung der Formel I nach Anspruch 1 mit verträglichem pharmazeutischen Träger.

7. Verbindungen der Formel I nach Anspruch 1 zur Anwendung in einem Verfahren zur Beeinflussung des Stoffwechsels von Kollagen und kollagenähnlichen Stoffen bzw. der Biosynthese von C1_{q}.

8. Verbindungen der Formel I nach Anspruch 1 zur Anwendung in einem Verfahren zur Behandlung von Störungen des Stoffwechsels von Kollagen und kollagenähnlichen Stoffen bzw. der Biosynthese von C1_{q}.

9. Verfahren zur Herstellung von Arzneimitteln zur Beeinflussung des Stoffwechsels von Kollagen und kollagenähnlichen Stoffen bzw. der Biosynthese von C1_{q}, dadurch gekennzeichnet, daß man in das Arzneimittel eine Verbindung der Formel I nach Anspruch 1 einverleibt.

## Patentansprüche (Patentansprüche für folgende(n) Vertragsstaat(en): ES, GR)

1. Verfahren zur Herstellung von Pyridin-2,4- und 2,5-dicarbonsäurediamide der Formel I 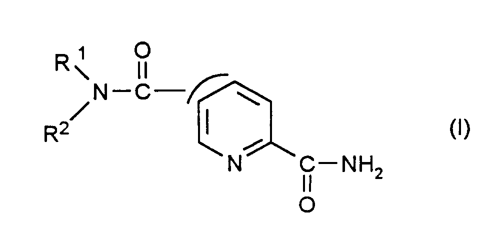 worin
R¹ C₁-C₁₂-Alkyl, bedeutet, welches unsubstituiert oder einfach oder im Falle der C₂-C₁₂-Alkyle auch mehrfach substituiert ist mit Phenyl, Hydroxy, Alkoxy, Alkoxycarbonyl, oder Dialkylamino, wobei die Alkylreste 1 bis 3 C-Atome aufweisen,
oder R¹ Phenyl bedeutet,
oder R¹ unter der Voraussetzung, daß R² H ist, Amino bedeutet, welches unsubstituiert ist oder mono-substituiert ist mit Methylcarbonyl
und
R² Wasserstoff bedeutet,
oder wobei die Reste R¹ und R² zusammen mit dem Stickstoffatom einen Rest der Formel 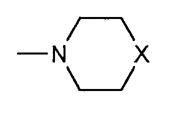 bilden,
worin
X O, CH₂ oder N-R³ bedeutet,
wobei
R³ Wasserstoff oder Methyl bedeutet, sowie die physiologisch verträglichen Salze,
dadurch gekennzeichnet, daß man eine Verbindung der Formel II' 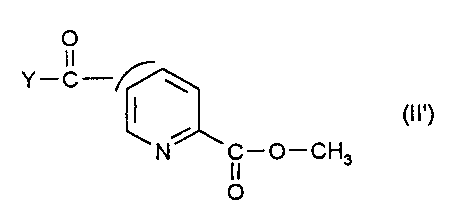 mit einer Verbindung der Formel III, 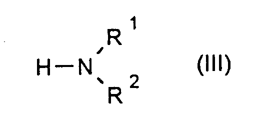 umsetzt, wobei R¹ und R² die zu Formel I angegebenen Bedeutungen haben und Y Halogen, insbesondere Chlor ist, und anschließend die entstehende Verbindung der Formel IV 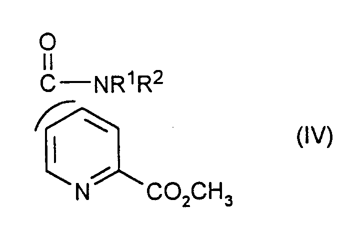 mit NH₃ in eine Verbindung der Formel I und in ihre physiologisch verträglichen Salze überführt.

2. Verbindungen nach Anspruch 1 zur Anwendung als Arzneimittel.

3. Verbindungen nach Anspruch 1 zur Inhibierung der Prolin- und Lysinhydroxylase.

4. Verbindungen nach Anspruch 1 zur Anwendung als Fibrosuppressiva und Immunsuppressiva.

5. Arzneimittel, enthaltend eine Verbindung der Formel I nach Anspruch 1 mit verträglichem pharmazeutischen Träger.

6. Verbindungen der Formel I nach Anspruch 1 zur Anwendung in einem Verfahren zur Beeinflussung des Stoffwechsels von Kollagen und kollagenähnlichen Stoffen bzw. der Biosynthese von C1_{q}.

7. Verbindungen der Formel I nach Anspruch 1 zur Anwendung in einem Verfahren zur Behandlung von Störungen des Stoffwechsels von Kollagen und kollagenähnlichen Stoffen bzw. der Biosynthese von C1_{q}.

8. Verfahren zur Herstellung von Arzneimitteln zur Beeinflussung des Stoffwechsels von Kollagen und kollagenähnlichen Stoffen bzw. der Biosynthese von C1_{q}, dadurch gekennzeichnet, daß man in das Arzneimittel eine Verbindung der Formel I nach Anspruch 1 einverleibt.

## Claims (Claims for the following Contracting State(s): AT, BE, CH, DE, DK, FR, GB, IT, LI, LU, NL, PT, SE)

1. A pyridine-2,4- or -2,5-dicarboxamide of the formula I 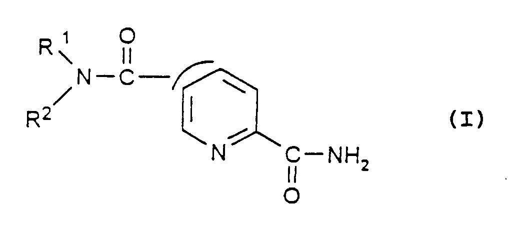 in which
R¹ is C₁-C₁₂-alkyl which is unsubstituted or substituted once or, in the case of the C₂-C₁₂-alkyls, also several times by phenyl, hydroxyl, alkoxy, alkoxycarbonyl, or dialkylamino, where the alkyl radicals have 1 to 3 carbon atoms,
or R¹ is phenyl,
or, provided that R² is H, R¹ is amino which is unsubstituted or monosubstituted by methyl
carbonyl and
R² is hydrogen,
or where the radicals R¹ and R² form, together with the nitrogen atom, a radical of the formula 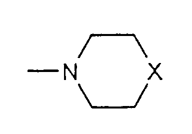 in which
X is O, CH₂ or N-R³,
where
R³ is hydrogen or methyl, and the physiologically tolerated salts

2. A process for the preparation of compounds of the formula I as claimed in claim 1, which comprises reacting a compound of the formula II' 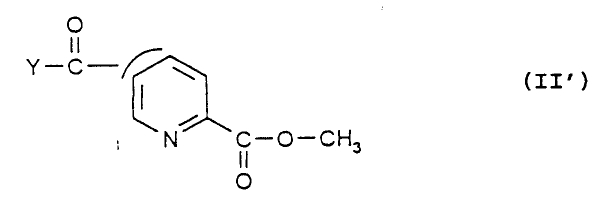 with a compound of the formula III 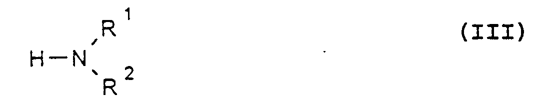 where R¹ and R² have the meanings indicated for formula I, and Y is halogen, especially chlorine, and subsequently converting the resulting compound of the formula IV 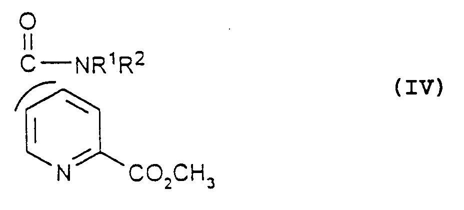 with NH₃ into a compound of the formula I and into its physiologically tolerated salts.

3. A compound as claimed in claim 1 for use as pharmaceutical.

4. A compound as claimed in claim 1 for the inhibition of proline hydroxylase and lysine hydroxylase.

5. A compound as claimed in claim 1 for use as fibrosuppressant and immunosuppressant.

6. A pharmaceutical containing a compound of the formula I as claimed in claim 1 with a compatible pharmaceutical vehicle.

7. A compound of the formula I as claimed in claim 1 for use in a method for influencing the metabolism of collagen and collagen-like substances and the biosynthesis of C1_{q}.

8. A compound of the formula I as claimed in claim 1 for use in a method for the treatment of disorders of the metabolism of collagen and collagen-like substances and of the biosynthesis of C1_{q}.

9. A process for the production of pharmaceuticals for influencing the metabolism of collagen and collagen-like substances and the biosynthesis of C1_{q}, which comprises incorporating a compound of the formula I as claimed in claim 1 in the pharmaceutical.

## Claims (Claims for the following Contracting State(s): ES, GR)

1. A process for the preparation of a pyridine-2,4- or -2,5-dicarboxamide of the formula I 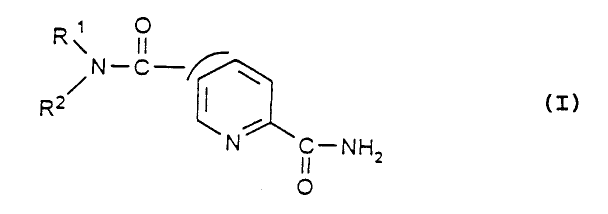 in which
R¹ is C₁-C₁₂-alkyl which is unsubstituted or substituted once or, in the case of the C₂-C₁₂-alkyls, also several times by phenyl, hydroxyl, alkoxy, alkoxycarbonyl, or dialkylamino, where the alkyl radicals have 1 to 3 carbon atoms,
or R¹ is phenyl,
or, provided that R² is H, R¹ is amino which is unsubstituted or monosubstituted by methylcarbonyl
and
R² is hydrogen,
or where the radicals R¹ and R² form, together with the nitrogen atom, a radical of the formula 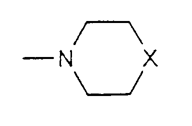
in which
X is O, CH₂ or N-R³,
where
R³ is hydrogen or methyl, and the physiologically tolerated salts,
which comprises reacting a compound of the formula II' 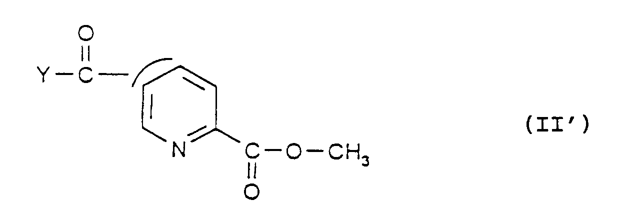 with a compound of the formula III 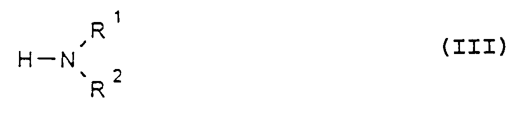 where R¹ and R² have the meanings indicated for formula I, and Y is halogen, especially chlorine, and subsequently converting the resulting compound of the formula IV 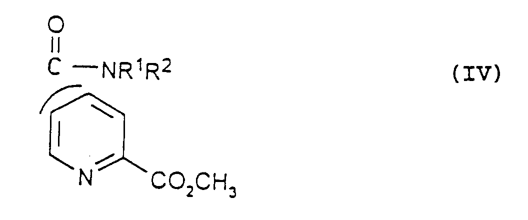 with NH₃ into a compound of the formula I and into its physiologically tolerated salts.

2. A compound as claimed in claim 1 for use as pharmaceutical.

3. A compound as claimed in claim 1 for the inhibition of proline hydroxylase and lysine hydroxylase.

4. A compound as claimed in claim 1 for use as fibrosuppressant and immunosuppressant.

5. A pharmaceutical containing a compound of the formula I as claimed in claim 1 with a compatible pharmaceutical vehicle.

6. A compound of the formula I as claimed in claim 1 for use in a method for influencing the metabolism of collagen and collagen-like substances and the biosynthesis of C1_{q}.

7. A compound of the formula I as claimed in claim 1 for use in a method for the treatment of disorders of the metabolism of collagen and collagen-like substances and of the biosynthesis of C1_{q}.

8. A process for the production of pharmaceuticals for influencing the metabolism of collagen and collagen-like substances and the biosynthesis of C1_{q}, which comprises incorporating a compound of the formula I as claimed in claim 1 in the pharmaceutical.

## Revendications (Revendications pour l'(les) Etat(s) contractant(s) suivant(s): AT, BE, CH, DE, DK, FR, GB, IT, LI, LU, NL, PT, SE)

1. Diamides d'acides pyridine-2,4- et -2,5-dicarboxyliques de formule I 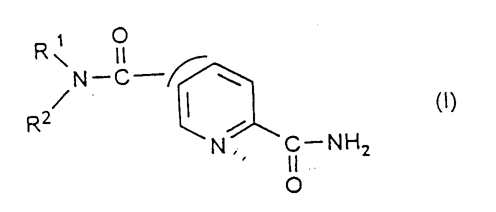 dans laquelle
R¹ désigne un groupe alkyle en C₁ à C₁₂, qui est non substitué ou que porte un seul substituant ou aussi, dans le cas des groupes alkyle en C₂ à C₁₂, plusieurs substituants choisis parmi :
phényle, hydroxy, alkoxy, alkoxycarbonyle,
ou dialkylamino, les restes alkyle ayant 1 à 3 atomes de carbone, ou bien R¹ désigne un groupe phényle,
ou bien, sous réserve que R² représente de l'hydrogène, R¹ désigne un groupe amino qui est non substitué ou mono-substitué avec un radical méthylcarbonyle
et
R² désigne de l'hydrogène
ou bien les restes R¹ et R² forment, conjointement avec l'atome d'azote, un reste de formule 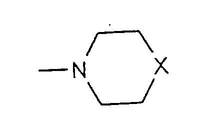 dans laquelle
X représente O, CH₂ ou un groupe N-R³,
dans lequel R³ désigne de l'hydrogène ou un groupe méthyle,
ainsi que les sels de ces composés acceptables du point de vue physiologique.

2. Procédé de préparation de composés de formule I suivant la revendication 1, caractérisé en ce qu'on fait réagir un composé de formule II' 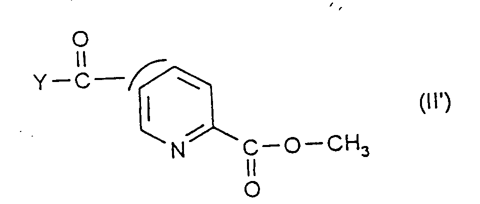 avec un composé de formule III 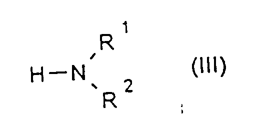 formules dans lesquelles R¹ et R² ont les définitions indiquées pour la formule I, et Y désigne un halogéne, en particulier le chlore
puis on transforme avec NH₃ le composé produit de formule IV 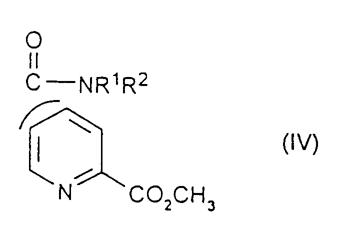 en un composé de formule I et en ses sels acceptables du point de vue physiologique.

3. Composés suivant la revendication 1, destinés à être utilisés comme médicament.

4. Composés suivant la revendication 1, destinés à inhiber la proline-hydroxylase et la lysine-hydroxylase.

5. Composés suivant la revendication 1, destinés à être utilisés comme fibrosuppresseurs et immunosuppresseurs.

6. Médicament contenant un composé de formule I suivant la revendication 1 et un véhicule acceptable du point de vue pharmaceutique.

7. Composés de formule I suivant la revendication 1, destinés à être utilisés dans un procédé pour influencer le métabolisme du collagène et de substances analogues au collagène et la biosynthèse du C1_{q}.

8. Composés de formule I suivant la revendication 1, destinés à être utilisés dans un procédé de traitement de troubles du métabolisme du collagène et de substances analogues au collagène et de la biosynthèse du C1_{q}.

9. Procédé de préparation de médicaments destinés à influencer le métabolisme du collagène et de substances analogues au collagène et la biosynthèse du C1_{q}, caractérisé en ce qu'on incorpore au médicament un composé de formule I suivant la revendication 1.

## Revendications (Revendications pour l'(les) Etat(s) contractant(s) suivant(s): ES, GR)

1. Procédé de préparation de diamides d'acides pyridine-2,4- et -2,5-dicarboxyliques de formule I 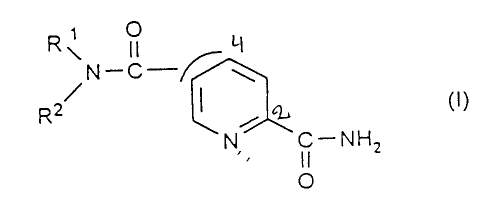 dans laquelle
R¹ désigne un groupe alkyle en C₁ à C₁₂, qui est non substitué ou qui porte un seul substituant ou aussi, dans le cas des groupes alkyle en C₂ à C₁₂, plusieurs substituants choisis parmi :
phényle, hydroxy, alkoxy, alkoxycarbonyle,
ou dialkylamino, les restes alkyle ayant 1 à 3 atomes de carbone, ou bien R¹ désigne un groupe phényle,
ou bien, sous réserve que R² représente de l'hydrogène, R¹ désigne un groupe amino qui est non substitué ou mono-substitué avec un radical méthylcarbonyle
et
R² désigne de l'hydrogène
ou bien les restes R¹ et R² forment, conjointement avec l'atome d'azote, un reste de formule 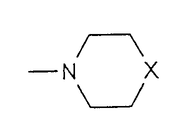 dans laquelle
X représente O, CH₂ ou un groupe N-R³,
dans lequel R³ désigne de l'hydrogène ou un groupe méthyle,
ainsi que leurs sels acceptables du point de vue physiologique,
caractérisé en ce qu'on fait réagir un composé de formule II' 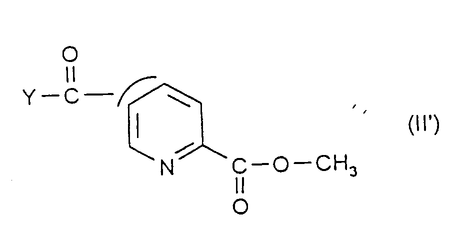 avec un composé de formule III 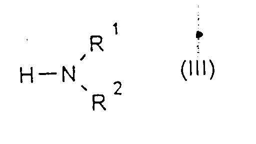 formule dans lesquelles R¹ et R² ont les définitions indiquées pour la formule I, et Y désigne un halogène, en particulier le chlore,
puis on transforme avec NH₃ le composé produit de formule IV 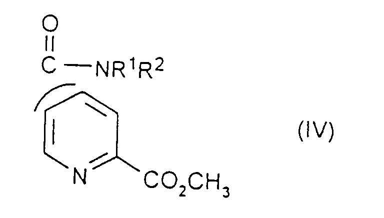 en un composé de formule I et en ses sels acceptables du point de vue physiologique.

2. Composés suivant la revendication 1, destinés à être utilisés comme médicament.

3. Composés suivant la revendication 1, destinés à inhiber la proline-hydroxylase et la lysine-hydroxylase.

4. Composés suivant la revendication 1, destinés à être utilisés comme fibrosuppresseurs et immunosuppresseurs.

5. Médicament contenant un composé de formule I suivant la revendication 1 et un véhicule acceptable du point de vue pharmaceutique.

6. Composés de formule I suivant la revendication 1, destinés à être utilisés dans un procédé pour influencer le métabolisme du collagène et de substances analogues au collagène et la biosynthèse du C1_{q}.

7. Composés de formule I suivant la revendication 1, destinés à être utilisés dans un procédé de traitement de troubles du métabolisme du collagène et de substances analogues au collagène et de la biosynthèse du C1_{q}.

8. Procédé de préparation de médicaments destinés à influencer le métabolisme du collagène et de substances analogues au collagène et la biosynthèse du C1_{q}, caractérisé en ce qu'on incorpore au médicament un composé de formule I suivant la revendication 1.
